(19) **Europäisches Patentamt / European Patent Office / Office européen des brevets**

(11) **EP 3 925 606 B1**

(12) **EUROPEAN PATENT SPECIFICATION**

(45) Date of publication and mention
of the grant of the patent:
**11.03.2026 Bulletin 2026/11**

(21) Application number: **20755904.8**

(22) Date of filing: **23.01.2020**

(51) International Patent Classification (IPC):
*A61K 31/381* (2006.01)    *A61P 25/14* (2006.01)
*A61P 25/16* (2006.01)    *A61K 9/70* (2006.01)
*A61K 47/06* (2006.01)    *A61K 47/10* (2017.01)
*A61K 47/22* (2006.01)    *A61K 47/32* (2006.01)

(52) Cooperative Patent Classification (CPC):
**A61K 9/7053; A61K 31/381; A61K 47/32;
A61P 25/14; A61P 25/16**

(86) International application number:
**PCT/JP2020/002302**

(87) International publication number:
**WO 2020/166298 (20.08.2020 Gazette 2020/34)**

(54) **ROTIGOTINE STABILIZATION METHOD**

VERFAHREN ZUR STABILISIERUNG VON ROTIGOTIN

PROCÉDÉ DE STABILISATION DE ROTIGOTINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.02.2019 JP 2019025151**

(43) Date of publication of application:
**22.12.2021 Bulletin 2021/51**

(73) Proprietor: **Hisamitsu Pharmaceutical Co., Inc.
Tosu-shi, Saga 841-0017 (JP)**

(72) Inventors:
• **KUROKAWA Takao
Tsukuba-shi, Ibaraki 305-0856 (JP)**
• **ARAKI Hiroyuki
Tosu-shi, Saga 841-0017 (JP)**

• **KOMATANI Kimiko
Tsukuba-shi, Ibaraki 305-0856 (JP)**

(74) Representative: **Grünecker Patent- und
Rechtsanwälte
PartG mbB
Leopoldstraße 4
80802 München (DE)**

(56) References cited:
**WO-A1-2012/084969    WO-A1-2019/124261
JP-A- 2012 140 407    JP-A- 2013 079 220**

Remarks:
The file contains technical information submitted
after the application was filed and not included in this
specification

EP 3 925 606 B1

# EP 3 925 606 B1

**Description**

[Technical Field]

The present invention relates to a rotigotine stabilization method, and more specifically to a method for stabilizing rotigotine and/or a pharmaceutically acceptable salt thereof in a rotigotine-containing patch comprising a backing layer and an adhesive agent layer, the adhesive agent layer containing the rotigotine and/or the pharmaceutically acceptable salt thereof.

[Background Art]

International Application Japanese-Phase Publication No. 2011-504902 (PTL 1) states that rotigotine is the international general name for the compound (-)-5,6,7,8-tetrahydro-6-[propyl-[2-(2-thienyl)ethyl]-amino] 1-naphthalenol, and has crystal polymorphs of type I and type II. Rotigotine is a D1/D2/D3 dopamine receptor agonist, and is mainly used for the treatment of symptoms of Parkinson's disease and restless legs syndrome.

For example, as a formulation for rotigotine administration, "Neupro (registered trademark) Patch" is commercially available in Japan and overseas. In addition, International Application Japanese-Phase Publication No. 2002-509878 (PTL 2) describes a transdermal therapeutic system comprising a backing layer inert to the components of a matrix, a self-adhesive matrix layer containing rotigotine, wherein the matrix is based on a non-aqueous, acrylate-based or silicone-based polymer adhesive having a solubility of 5% (w/w) or more for rotigotine. Moreover, International Application Japanese-Phase Publication No. 2015-503541 (PTL 3) describes a transdermal therapeutic system comprising a backing layer that is impermeable to active substances, and a matrix layer including a pressure-sensitive adhesive, a drug, and particles of cross-linked polyvinylpyrrolidone, wherein the drug is rotigotine and the pressure-sensitive adhesive is a silicone polymer.

In addition, as a formulation for rotigotine administration using a rubber-based adhesive agent, Japanese Unexamined Patent Application Publication No. 2014-177428 (PTL 4) describes a transdermal absorption-type patch formulation comprising a support, and a drug-containing layer which includes a rubber-based adhesive agent containing a rosin-based resin and a rubber-based adhesive component, and which includes rotigotine or a pharmaceutically acceptable salt thereof, and Japanese Unexamined Patent Application Publication No. 2013-079220 (PTL 5) describes a transdermal absorption-type patch comprising a support, and a drug-containing layer including a rubber-based adhesive agent, rotigotine or a salt thereof, and a production inhibitor of rotigotine decomposition products, for example.

Moreover, as a formulation for rotigotine administration, International Application Japanese-Phase Publication No. 2006-513195 (PTL 6) and International Application Japanese-Phase Publication No. 2012-504609 (PTL 7) state that rotigotine is incorporated in an amorphous form, and International Application Japanese-Phase Publication No. 2013-515041 (PTL 8) describes a rotigotine stabilization method comprising the step of providing a solid dispersion containing polyvinylpyrrolidone and amorphous rotigotine using polyvinylpyrrolidone (non-cross-linked) in a specific weight ratio with respect to rotigotine, for example.

Moreover, International Application Japanese-Phase Publication No. 2017-515871 (PTL 9) describes a method for producing a transdermal absorption formulation by mixing rotigotine and an antioxidant at a specific weight ratio for the purpose of preventing the precipitation of rotigotine crystals.

WO 2012/084969 A1 (PTL 10) discloses a transdermal patch comprising a backing layer and an adhesive layer, the adhesive layer containing rotigotine, a styrene-based thermoplastic elastomer and cross-linked polyvinylpyrrolidone.

[Citation List]

[Patent Literature]

[PTL 1] International Application Japanese-Phase Publication No. 2011-504902
[PTL 2] International Application Japanese-Phase Publication No. 2002-509878
[PTL 3] International Application Japanese-Phase Publication No. 2015-503541
[PTL 4] Japanese Unexamined Patent Application Publication No. 2014-177428
[PTL 5] Japanese Unexamined Patent Application Publication No. 2013-079220
[PTL 6] International Application Japanese-Phase Publication No. 2006-513195
[PTL 7] International Application Japanese-Phase Publication No. 2012-504609
[PTL 8] International Application Japanese-Phase Publication No. 2013-515041
[PTL 9] International Application Japanese-Phase Publication No. 2017-515871

[PTL 10] WO 2012/084969 A1

[Summary of Invention]

[Technical Problem]

Here, the present inventors have made further studies on rotigotine-containing patches containing rotigotine and/or a pharmaceutically acceptable salt thereof. As a result, the present inventors have found that in the case of using a silicone-based adhesive base agent or an acrylic-based adhesive agent, which has heretofore been used frequently in combination with rotigotine, or simply using a rubber-based adhesive agent such as polyisobutylene as the adhesive base agent to be incorporated in the adhesive agent layer of a patch, the long-term stability of the rotigotine and/or the pharmaceutically acceptable salt thereof may be insufficient under particularly harsh conditions, and that a higher level of long-term stability is required.

The present invention has been made in view of the above-described problem, and aims to provide a rotigotine stabilization method that enables high-level stabilization of rotigotine and/or a pharmaceutically acceptable salt thereof in an adhesive agent layer of a patch containing a styrene-based thermoplastic elastomer.

[Solution to Problem]

The present inventors have made earnest studies to achieve the above object, and have found that, in a rotigotine-containing patch which is a patch comprising a backing layer and an adhesive agent layer and containing, in the adhesive agent layer, at least one selected from the group consisting of rotigotine and pharmaceutically acceptable salts thereof (hereinafter referred to as "rotigotine and/or a/the pharmaceutically acceptable salt thereof" when appropriate) and a styrene-based thermoplastic elastomer, the rotigotine and/or the pharmaceutically acceptable salt thereof can be stabilized at a high level for a long period of time even under harsh conditions, by further incorporating cross-linked polyvinylpyrrolidone in the adhesive agent layer in a particular content range. As a result, the present invention has been completed.

Specifically, the rotigotine stabilization method of the present invention is

a method for stabilizing rotigotine and/or a pharmaceutically acceptable salt thereof in a rotigotine-containing patch comprising a backing layer and an adhesive agent layer, the adhesive agent layer containing the rotigotine and/or the pharmaceutically acceptable salt thereof and a styrene-based thermoplastic elastomer,
the method characterized in that it comprises further incorporating cross-linked polyvinylpyrrolidone in the adhesive agent layer in a content of 3 to 25% by mass in the adhesive agent layer, and an alicyclic saturated hydrocarbon resin in the adhesive agent layer, with the proviso that a patch prepared according to the further method described in claim 1 is excluded.

In the rotigotine stabilization method of the present invention, a content of the rotigotine and/or the pharmaceutically acceptable salt thereof in the adhesive agent layer is preferably 5 to 15% by mass in terms of rotigotine free form.

In addition, in the rotigotine stabilization method of the present invention, a mass ratio of a content of the rotigotine and/or the pharmaceutically acceptable salt thereof in terms of rotigotine free form and a content of the cross-linked polyvinylpyrrolidone in the adhesive agent layer (the content of the rotigotine and/or the pharmaceutically acceptable salt thereof in terms of rotigotine free form : the content of the cross-linked polyvinylpyrrolidone) is 15:3 to 5:25.

Moreover, in the rotigotine stabilization method of the present invention, it is preferable to further incorporate an imidazole-based antioxidant in the adhesive agent layer, and in addition, more preferable that a content of the imidazole-based antioxidant in the adhesive agent layer be 0.05 to 2% by mass.

Moreover, in the rotigotine stabilization method of the present invention, it is preferable to further incorporate an aliphatic alcohol in the adhesive agent layer.

[Advantageous Effects of Invention]

According to the present invention, it is possible to provide a rotigotine stabilization method that enables high-level stabilization of rotigotine and/or a pharmaceutically acceptable salt thereof in an adhesive agent layer of a patch containing a styrene-based thermoplastic elastomer.

[Description of Embodiments]

Hereinafter, the present invention is described in detail based on preferred embodiments thereof. The rotigotine stabilization method of the present invention is

a method for stabilizing rotigotine and/or a pharmaceutically acceptable salt thereof in a rotigotine-containing patch comprising a backing layer and an adhesive agent layer, the adhesive agent layer containing the rotigotine and/or the pharmaceutically acceptable salt thereof and a styrene-based thermoplastic elastomer,
the method comprising further incorporating cross-linked polyvinylpyrrolidone in the adhesive agent layer in a content of 3 to 25% by mass in the adhesive agent layer, and an alicyclic saturated hydrocarbon resin in the adhesive agent layer, with the proviso that a patch prepared according to the further method described in claim 1 is excluded.

The rotigotine-containing patch according to the present invention comprises a backing layer and an adhesive agent layer. The backing layer is not particularly limited as long as it can support the adhesive agent layer to be described later, and a known backing layer for a patch can be appropriately employed. Examples of the material of the backing layer according to the present invention include polyolefins such as polyethylene and polypropylene; ethylene-vinyl acetate copolymer, vinyl acetate-vinyl chloride copolymer, polyvinyl chloride, and the like; polyamides such as nylon; polyesters such as polyethylene terephthalate (PET), polybutylene terephthalate, and polyethylene naphthalate; cellulose derivatives; and synthetic resins such as polyurethane, and metals such as aluminum. Among these, polyesters and polyethylene terephthalate are preferable from the viewpoint of non-adsorbability and non-permeability of drugs. Examples of the form of the backing layer include films; sheet-shaped objects such as sheets, sheet-shaped porous bodies, and sheet-shaped foams; fabrics such as woven fabrics, knitted fabrics, and nonwoven fabrics; foils; and laminates thereof. In addition, the thickness of the backing layer is not particularly limited, but is preferably in the range of 5 to 1000 $\mu$m from the viewpoints of ease of operation of applying the patch and ease of production.

The rotigotine-containing patch according to the present invention may further comprise a release liner on the surface of the adhesive agent layer opposite to the backing layer. Examples of such a release liner include polyolefins such as polyethylene and polypropylene; ethylene-vinyl acetate copolymer, vinyl acetate-vinyl chloride copolymer, polyvinyl chloride, and the like; polyamides such as nylon; polyesters such as polyethylene terephthalate; cellulose derivatives; and films and sheets made of materials such as synthetic resins including polyurethane, aluminum, and paper, and laminates thereof. Preferably, these release liners have been subjected to a release treatment using a silicone-containing compound coat, a fluorine-containing compound coat, or the like on the surface to be in contact with the adhesive agent layer so as to enable easy release from the adhesive agent layer.

<Rotigotine and Pharmaceutically Acceptable Salt Thereof>

The adhesive agent layer according to the present invention contains at least one selected from the group consisting of rotigotine and pharmaceutically acceptable salts thereof (rotigotine and a pharmaceutically acceptable salt thereof) as a drug. In the present invention, the form of the rotigotine contained in the adhesive agent layer may be a free form or a pharmaceutically acceptable salt thereof, may be a pharmaceutically acceptable salt of rotigotine that has been desalted into a free form in the formulation during production and/or after production, or may be one of these or a mixture of two or more thereof. Examples of the pharmaceutically acceptable salt of rotigotine include acid addition salts, and examples of the acid addition salts include hydrochloric acid, sulfuric acid, nitric acid, phosphoric acid, phosphorous acid, hydrobromic acid, maleic acid, malic acid, ascorbic acid, tartaric acid, lauric acid, stearic acid, palmitic acid, oleic acid, myristic acid, lauryl sulfate, linolenic acid, and fumaric acid. Among these, the adhesive agent layer according to the present invention preferably contains rotigotine in a free form.

In the rotigotine stabilization method of the present invention, the content of the rotigotine and/or the pharmaceutically acceptable salt thereof contained in the adhesive agent layer (the content of rotigotine, the content of the pharmaceutically acceptable salt of rotigotine, or the total content thereof if both of them are contained, hereinafter the same) is, in terms of rotigotine free form, preferably 5 to 15% by mass, more preferably 7 to 15% by mass, further preferably 7 to 12% by mass, and even further preferably 8 to 10% by mass relative to the total mass of the adhesive agent layer (in this description, "the total mass of the adhesive agent layer" indicates the total mass of the adhesive agent layer in the patch after production (after the incorporation of the below-described cross-linked polyvinylpyrrolidone and alicyclic saturated hydrocarbon resin and, optionally, imidazole-based antioxidant and aliphatic alcohol)

When the content of the rotigotine and/or the pharmaceutically acceptable salt thereof is less than the lower limit, the skin permeability of the rotigotine tends to decrease. Meanwhile, when the upper limit is exceeded, there is a tendency that the rotigotine and/or the pharmaceutically acceptable salt thereof is stabilized without incorporating cross-linked polyvinylpyrrolidone in the adhesive agent layer, and in addition a tendency that crystals of the rotigotine and/or the pharmaceutically acceptable salt thereof are precipitated, an amorphous form is formed, and the adhesive force of the adhesive agent layer is easily reduced.

<Styrene-Based Thermoplastic Elastomer>

The adhesive agent layer according to the present invention contains a styrene-based thermoplastic elastomer as an adhesive base agent. The styrene-based thermoplastic elastomer according to the present invention is a thermoplasticity-exhibiting styrene-based elastomer which exhibits fluidity by softening when heated, and which returns to a rubber-like elastic body when cooled. Among these, a styrene-based block copolymer is preferred from the viewpoint of sufficient tackiness impartment and stability over time.

Specific examples of the styrene-based block copolymer include styrene-butadiene block copolymer, styrene-butadiene-styrene block copolymer (SBS), styrene-isoprene block copolymer, styrene-isoprene-styrene block copolymer (SIS), styrene-ethylene/butylene block copolymer, styrene-ethylene/butylene-styrene block copolymer, styrene-ethylene/propylene block copolymer, styrene-ethylene/propylene-styrene block copolymer, styrene-isobutylene block copolymer, styrene-isobutylene-styrene block copolymer, and the like, and one of these may be used alone, or two or more may be used in combination. Note that, in the above, "ethylene/butylene" indicates a copolymer block of ethylene and butylene, and "ethylene/propylene" indicates a copolymer block of ethylene and propylene. Among these, the styrene-based thermoplastic elastomer according to the present invention is more preferably a styrene-isoprene-styrene block copolymer.

The styrene-isoprene-styrene block copolymer has a viscosity average molecular weight of preferably 30,000 to 2,500,000, and more preferably 100,000 to 1,700,000. When the viscosity average molecular weight is less than the lower limit, the formulation properties of the patch (particularly the cohesive force of the adhesive agent layer) tend to decrease. Meanwhile, when the upper limit is exceeded, there is a tendency that the compatibility with other components contained in the adhesive agent layer is reduced, making it difficult to produce a patch.

In the rotigotine stabilization method of the present invention, the content of the styrene-based thermoplastic elastomer contained in the adhesive agent layer is preferably 5 to 80% by mass, preferably 5 to 50% by mass, more preferably 10 to 40% by mass, and further preferably 10 to 30% by mass relative to the total mass of the adhesive agent layer. When the content of the styrene-based thermoplastic elastomer is less than the lower limit, the cohesive force, shape retainability, and the like of the adhesive agent layer tend to decrease. Meanwhile, when the upper limit is exceeded, there is a tendency that the cohesive force of the adhesive agent layer excessively increases, so that the adhesive force of the adhesive agent layer decreases or the compatibility decreases.

<Cross-Linked Polyvinylpyrrolidone>

In the rotigotine stabilization method of the present invention, cross-linked polyvinylpyrrolidone (also referred to as "cross-linked PVP") is incorporated in the adhesive agent layer containing at least the rotigotine and/or the pharmaceutically acceptable salt thereof and the styrene-based thermoplastic elastomer.

Examples of the cross-linked polyvinylpyrrolidone according to the present invention include a cross-linked N-vinylpyrrolidone polymer. The N-vinylpyrrolidone polymer may be a homopolymer or a copolymer, and examples thereof include a homopolymer of N-vinylpyrrolidone and a copolymer of N-vinylpyrrolidone and a polyfunctional monomer. Among these, the cross-linked polyvinylpyrrolidone according to the present invention is preferably a cross-linked homopolymer of 1-vinyl-2-pyrrolidone (also referred to as "crospovidone") . As crospovidone, commercially available ones may be used, such as Kollidon CL and Kollidon CL-M (manufactured by BASF Japan Ltd.); and Polyplasdone XL, Polyplasdone XL-10, and Polyplasdone INF-10 (manufactured by ISP Japan Ltd.).

The amount of the cross-linked polyvinylpyrrolidone to be incorporated in the adhesive agent layer needs to be such an amount that the content relative to the total mass of the adhesive agent layer is 3 to 25% by mass. When the content of the cross-linked polyvinylpyrrolidone in the adhesive agent layer is less than the lower limit, it leads to a failure to exhibit a sufficient rotigotine stabilization effect of long-term stabilization of the rotigotine and/or the pharmaceutically acceptable salt thereof under particularly harsh conditions. Meanwhile, when the upper limit is exceeded, the skin permeability of the rotigotine decreases or the compatibility in an adhesive agent layer composition decreases during production, making the production difficult. The content of the cross-linked polyvinylpyrrolidone in the adhesive agent layer is more preferably 3 to 20% by mass, and further preferably 3 to 15% by mass, and even further preferably 4 to 15% by mass from a similar viewpoint.

In addition, in the rotigotine stabilization method of the present invention, the mass ratio of the content of the rotigotine and/or the pharmaceutically acceptable salt thereof in terms of rotigotine free form and the content of the cross-linked polyvinylpyrrolidone in the adhesive agent layer (the content of the rotigotine and/or the pharmaceutically acceptable salt thereof in terms of rotigotine free form : the content of the cross-linked polyvinylpyrrolidone) is preferably 15:3 to 5:25, more preferably 15:3 to 5:20, and further preferably 15:3 to 5:15. When the content of the cross-linked polyvinylpyrrolidone relative to the rotigotine and/or the pharmaceutically acceptable salt thereof is less than the lower limit, crystals of the rotigotine and/or the pharmaceutically acceptable salt thereof tend to precipitate. Meanwhile, when the upper limit is exceeded, it tends to be difficult to achieve good skin permeability of the rotigotine.

Moreover, the amount of the cross-linked polyvinylpyrrolidone to be incorporated in the adhesive agent layer is also preferably such that, when the content relative to the total mass of the adhesive agent layer is less than 4% by mass (preferably 3.95% by mass or less), the mass ratio in the adhesive agent layer (the content of the rotigotine and/or the pharmaceutically acceptable salt thereof in terms of rotigotine free form : the content of the cross-linked polyvinylpyrrolidone) is 15:3 to 12:3 or 7:3 to 5:15 (more preferably 7:3 to 5:15 from the viewpoint of suppressing the precipitation of crystals of the rotigotine and/or the pharmaceutically acceptable salt thereof and the formation of an amorphous form, and the like.

<Antioxidant>

In the rotigotine stabilization method of the present invention, it is preferable to further incorporate an antioxidant in the adhesive agent layer. In the present invention, by incorporating the antioxidant in the adhesive agent layer in addition to the cross-linked polyvinylpyrrolidone, the stability over time tends to be achieved at a higher level.

Examples of the antioxidant include imidazole-based antioxidants (such as 2-mercaptobenzimidazole), and one of these may be used alone or two or more may be used in combination. Among these, the antioxidant is particularly preferably 2-mercaptobenzimidazole.

Here, the content of the antioxidant to be incorporated in the adhesive agent layer (in the case of a combination of two or more kinds, the total content of these, hereinafter the same; preferably the content of an imidazole-based antioxidant, and more preferably the content of 2-mercaptobenzimidazole) is preferably 0.05 to 2% by mass, more preferably 0.05 to 1.5% by mass, and further preferably 0.05 to 1.0% by mass relative to the total mass of the adhesive agent layer. When the content of the antioxidant is less than the lower limit, the further stabilization effect by the antioxidant tends not to be exhibited sufficiently. Meanwhile, when the upper limit is exceeded, the physical properties of the adhesive agent layer such as its adhesiveness may decrease.

<Petroleum-Based Resin · Terpene-Based Resin>

In the rotigotine stabilization method of the present invention, a petroleum-based resin, namely an alicyclic saturated hydrocarbon resin, is incorporated the adhesive agent layer. In the rotigotine stabilization method of the present invention, by incorporating the petroleum-based resin in the adhesive agent layer, a high level of skin permeability is achieved, and in addition, the production of rotigotine analog substances is suppressed, so that the stability over time tends to be further improved.

(Petroleum-Based Resin)

Examples of the petroleum-based resin include C5-based synthetic petroleum resins (such as a copolymer of at least two of isoprene, cyclopentadiene, 1,3-pentadiene, and 1-pentene; a copolymer of at least two of 2-pentene and dicyclopentadiene; and a 1,3-pentadiene-based resin), C9-based synthetic petroleum resins (such as a copolymer of at least two of indene, styrene, methylindene, and α-methylstyrene), and a dicyclopentadiene-based synthetic petroleum resin (a copolymer with isoprene and/or 1,3-pentadiene mainly composed of dicyclopentadiene). In addition, from the viewpoint of another classification, examples include alicyclic petroleum resins (such as alicyclic saturated hydrocarbon resins), alicyclic hydrogenated petroleum resins, aliphatic petroleum resins (such as aliphatic hydrocarbon resins), aliphatic hydrogenated petroleum resins, and aromatic petroleum resins, and more specific examples include Arkon P-70, Arkon P-85, Arkon P-90, Arkon P-100, Arkon P-115, Arkon P-125 (these are trade names, manufactured by Arakawa Chemical Industries, Ltd.), and Escorez 8000 (trade name, manufactured by Esso Petrochemical Co. Ltd.). As the petroleum-based resin according to the present invention, an alicyclic saturated hydrocarbon resin is used. with an alicyclic saturated hydrocarbon resin, suitable adhesion tc the skin is easily obtained, the feeling of use is good due to little odor and the like, and the production of rotigotine analog substances is further suppressed.

In the present invention, the alicyclic saturated hydrocarbon resin refers to a resin that is a homopolymer or copolymer of alicyclic saturated hydrocarbon monomers. The alicyclic saturated hydrocarbon resin has a weight average molecular weight of preferably 1,000 to 1,500, and more preferably 1,200 to 1,400.

(Terpene-Based Resin) (not according to the invention)

Examples of the terpene-based resin include pinene polymers (such as α-pinene polymers and β-pinene polymers), terpene polymers, dipentene polymers, terpene-phenol polymers, aromatic modified terpene polymers, and pinene-phenol copolymers. More specific examples include YS RESIN (such as YS RESIN PXN (1150N, 300N), YS RESIN PX1000, YS RESIN T0125, and YS RESIN T0105), CLEARON P105, CLEARON M115, CLEARON K100 (these are trade names, manufactured by YASUHARA CHEMICAL CO., LTD.), and Tamanol 901 (trade name, manufactured by Arakawa Chemical Industries, Ltd.), and one of these may be used alone, or two or more may be used in combination. Among these, the terpene-based resin is more preferably a pinene polymer from the viewpoint that suitable adhesion to the skin is easily obtained, and the feeling of use is good due to little odor and the like.

In the present invention, the content of the petroleum-based resin be incorporated in the adhesive agent layer is preferably 5 to 80% by mass, more preferably 10 to 70% by mass, further preferably 10 to 60% by mass, and particularly preferably 20 to 60% by mass relative to the total mass of the adhesive agent layer. When the content of the petroleum-based resin is less than the lower limit, there is a tendency that the adhesive force of the adhesive agent layer and the adhesion to the skin decrease, and the effect of suppressing the production of rotigotine analog substances is not sufficiently exhibited. Meanwhile, when the upper limit is exceeded, the transdermal absorbability of the drug and the shape retainability of the adhesive agent layer tend to decrease.

<Aliphatic Alcohol>

In the rotigotine stabilization method of the present invention, it is preferable to further incorporate an aliphatic alcohol in the adhesive agent layer. In the present invention, the aliphatic alcohol refers to a saturated or unsaturated, linear or branched, monohydric or dihydric or higher aliphatic alcohol.

The aliphatic alcohol according to the present invention is preferably monohydric. In addition, the number of carbon atoms of the aliphatic alcohol according to the present invention is preferably 3 to 23 carbon atoms and more preferably 12 to 23 carbon atoms, further preferably 17 to 23 and particularly preferably 19 to 21 from the viewpoint of stability over time, and particularly preferably 12 to 20 from the viewpoint of skin permeability. When the number of carbon atoms of the aliphatic alcohol is less than the lower limit, the boiling point is low and thus it is difficult to keep a constant content in the formulation, so that the stability over time tends to decrease. Meanwhile, when the upper limit is exceeded, the compatibility with the adhesive base agent and other components tends to decrease.

Examples of the aliphatic alcohol according to the present invention include isopropanol, hexyl alcohol, lauryl alcohol, myristyl alcohol, cetyl alcohol, stearyl alcohol, isostearyl alcohol, octyldodecanol, oleyl alcohol, linolenyl alcohol, and hexyldecanol, and one of these may be used alone, or two or more may be used in combination. Among these, the aliphatic alcohol according to the present invention is particularly preferably at least one selected from the group consisting of octyldodecanol and lauryl alcohol from the viewpoint that the skin permeability of the rotigotine tends to be particularly improved, in addition to the viewpoints of stability over time and compatibility described above.

In the present invention, the content of the aliphatic alcohol to be incorporated in the adhesive agent layer is, in the case of two or more kinds, preferably 1 to 15% by mass, more preferably 1 to 10% by mass, further preferably 2 to 7% by mass, and particularly preferably 3 to 7% by mass in total relative to the total mass of the adhesive agent layer. When the content of the aliphatic alcohol is less than the lower limit, the skin permeability of the rotigotine tends to decrease. Meanwhile, when the upper limit is exceeded, the compatibility with the adhesive base agent and other components tends to decrease.

<Additional Components>

As long as the effects of the present invention are not impaired, the adhesive agent layer of the rotigotine-containing patch being a target of the rotigotine stabilization method of the present invention may further contain an additional drug other than rotigotine and a pharmaceutically acceptable salt thereof; an additional adhesive base agent other than the styrene-based thermoplastic elastomer; an additional tackifier other than the petroleum-based resin; an additional absorption promoter other than the aliphatic alcohol; and additives such as an adsorbent, desalting agent, plasticizer, solubilizer, filler, stabilizer, and preservative.

(Additional Drug)

Examples of the additional drug other than the rotigotine and the pharmaceutically acceptable salt thereof include nonsteroidal anti-inflammatory analgesics (such as diclofenac, indomethacin, ketoprofen, felbinac, loxoprofen, ibuprofen, flurbiprofen, tiaprofen, acemetacin, sulindac, etodolac, tolmetin, piroxicam, meloxicam, ampiroxicam, naproxen, azapropazone, methyl salicylate, glycol salicylate, valdecoxib, celecoxib, rofecoxib, and amfenac), antipyretic analgesics (such as acetaminophen), antihistamines (such as diphenhydramine, chlorpheniramine, mequitazine, and homochlorcyclizine), antihypertensives (such as diltiazem, nicardipine, nilvadipine, metoprolol, bisoprolol, and trandolapril), antiparkinsonian drugs (such as pergolide, ropinirole, bromocriptine, and selegiline), bronchodilators (such as tulobuterol, isoproterenol, and salbutamol), antiallergic agents (such as ketotifen, loratadine, azelastine, terfenadine, cetirizine, and acitazanolast), local anesthetics (such as lidocaine and dibucaine), neuropathic pain medications (such as pregabalin), non-narcotic analgesics (buprenorphine, tramadol, pentazocine), anesthetic analgesics (such as morphine, oxycodone, and fentanyl), agents for urinary organs (such as oxybutynin and tamsulosin), psychotropic agents (such as promazine and chlorpromazine), steroid hormones (such as estradiol, progesterone, norethisterone, cortisone, and hydrocortisone), antidepressants (such as sertraline, fluoxetine, paroxetine, and citalopram), anti-dementia drugs (such as donepezil, rivastigmine, and galantamine), antipsychotics (such as risperidone and olanzapine), central nervous system stimulants (such as methylphenidate), osteoporosis medications (such as raloxifene and alendronate), breast cancer prevention drugs (such as tamoxifen), anti-obesity drugs (such as mazindol and sibutramine), insomnia improving drugs (such as melatonin), and antirheumatic drugs (such as actarit), and one of these may be used alone, or two or more may be used in combination.

In the present invention, consider the case where these additional drugs are further contained in the adhesive agent layer. The content thereof is, in the case of two or more kinds, preferably 10% by mass or less in total relative to the total mass of the adhesive agent layer.

(Additional Adhesive Base Agent)

Examples of the additional adhesive base agent other than the styrene-based thermoplastic elastomer include rubber-based adhesive base agents other than the styrene-based thermoplastic elastomer, acrylic-based adhesive base agents, and silicone-based adhesive base agents, and one of these may be used alone, or two or more may be used in combination.

Examples of the rubber-based adhesive base agents other than the styrene-based thermoplastic elastomer include isoprene rubber, polyisobutylene (PIB), polybutene, and the like, and one of these may be used alone, or two or more may be used in combination. Among these, it is preferable to use polyisobutylene from the viewpoint that the tackiness and cohesive force of the adhesive agent layer tend to be further improved. In that case, it is more preferable that the mass ratio of the styrene-based thermoplastic elastomer (more preferably styrene-isoprene-styrene block copolymer) to the polyisobutylene (the mass of the styrene-based thermoplastic elastomer : the mass of PIB) be 1:2 to 30:1 (further preferably in the range of 1:1 to 10:1).

Examples of the acrylic-based adhesive base agents are listed in "Japanese Pharmaceutical Excipients Directory 2016 (edited by International Pharmaceutical Excipients Council Japan)" as adhesive agents, such as acrylic acid·octyl acrylate ester copolymer, 2-ethylhexyl acrylate·vinyl pyrrolidone copolymer, acrylate ester·vinyl acetate copolymer, 2-ethylhexyl acrylate·2-ethylhexyl methacrylate·dodecyl methacrylate copolymer, methyl acrylate·2-ethylhexyl acrylate copolymer resin, 2-ethylhexyl acrylate·methyl acrylate·acrylic acid·glycidyl methacrylate copolymer, 2-ethylhexyl acrylate·vinyl acetate·hydroxyethyl acrylate·glycidyl methacrylate copolymer, 2-ethylhexyl acrylate·diacetone acrylamide·acetoacetoxyethyl methacrylate·methyl methacrylate copolymer, ethyl acrylate·methyl methacrylate copolymer, acrylic-based polymer contained in an alkanolamine solution of acrylic resin, and the like, and one of these may be used alone, or two or more may be used in combination.

Examples of the silicone-based adhesive base agents include polydimethylsiloxane (such as the polymer represented by MQ in the representation by ASTM D-1418), polymethylvinylsiloxane (such as the polymer represented by VMQ in the representation by ASTM D-1418), polymethylphenylsiloxane (such as the polymer represented by PVMQ in the representation by ASTM D-1418), and the like, and one of these may be used alone, or two or more may be used in combination.

In the present invention, consider the case where these additional adhesive base agents are further contained. The content thereof is, in the case of two or more kinds, preferably 10% by mass or less in total relative to the total mass of the adhesive agent layer.

(Additional Tackifier)

In the rotigotine-containing patch according to the present invention, the tackifier is blended mainly for the purpose of increasing the tackiness of the adhesive base agent. Examples of the additional tackifier include tackifier resins other than the petroleum-based resin, such as terpene-based resins, rosin-based resins, phenol-based resins, and xylene-based resins, and one of these may be used alone, or two or more may be used in combination. In the present invention, consider the case where these additional tackifiers are further contained. The content thereof is, in the case of two or more kinds, preferably 10% by mass or less in total relative to the total mass of the adhesive agent layer.

(Additional Absorption Promoter (Transdermal Absorption Promoter))

Examples of the additional absorption promoter include those having an effect of promoting the transdermal absorption of drugs other than the aliphatic alcohol, such as fatty acids having 6 to 20 carbon atoms, fatty acid esters, fatty acid amides, or aliphatic alcohol ethers; aromatic organic acids; aromatic alcohols; aromatic organic acid esters or ethers; POE hydrogenated castor oils; lecithins; phospholipids; soybean oil derivatives; and triacetin, and one of these may be used alone, or two or more may be used in combination. In the present invention, consider the case where these absorption promoters are further contained. The content thereof is, in the case of two or more kinds, preferably 10% by mass or less in total relative to the total mass of the adhesive agent layer.

(Additives)

[Adsorbent]

Examples of the adsorbent include hygroscopic inorganic and/or organic substances, and more specific examples thereof include minerals such as talc, kaolin, and bentonite; silicon compounds such as fumed silica (such as AEROSIL (registered trademark)) and hydrous silica; metal compounds such as zinc oxide and dried aluminum hydroxide gel; weak acids such as lactic acid and acetic acid; sugars such as dextrin; and polymers such as polyvinylpyrrolidone (non-cross-linked PVP), aminoalkyl methacrylate copolymer, carboxyvinyl polymer, and butyl methacrylate methyl methacrylate copolymer, and one of these may be used alone, or two or more may be used in combination. In the present invention, consider the case where these adsorbents are further contained in the adhesive agent layer. The content thereof is, in the case of two or more kinds, preferably 10% by mass or less in total relative to the total mass of the adhesive agent layer.

[Desalting Agent]

The desalting agent is blended mainly for the purpose of converting all or a part of the basic drug into a free form. Such a desalting agent is not particularly limited, but is preferably, for example, a basic substance, and more preferably a metal ion-containing desalting agent or a basic nitrogen atom-containing desalting agent in the case of blending an acid addition salt of a drug as the drug to obtain a formulation containing a free form drug. Examples of the metal ion-containing desalting agent include sodium acetate (including anhydrous sodium acetate), sodium hydroxide, potassium hydroxide, magnesium hydroxide, sodium hydrogen carbonate, potassium hydrogen carbonate, sodium citrate, sodium lactate, and the like, and one of these may be used alone, or two or more may be used in combination. Note that the adhesive agent layer according to the present invention may further contain a compound derived from the basic drug and the desalting agent (for example, when rotigotine hydrochloride and sodium acetate are combined, sodium chloride). In the present invention, consider the case where these desalting agents and compounds derived from basic drugs and desalting agents are further contained in the adhesive agent layer. The content thereof is, in the case of two or more kinds, preferably 10% by mass or less in total relative to the total mass of the adhesive agent layer.

[Plasticizer]

The plasticizer is blended mainly for the purpose of adjusting the adhesive properties of the adhesive agent layer, flow characteristics in the production of the adhesive agent layer, transdermal absorption characteristics of the drug, and the like. Examples of such a plasticizer include silicone oils; petroleum-based oils such as paraffinic process oils, naphthenic process oils, and aromatic process oils; squalane and squalene; vegetable-based oils such as olive oil, camellia oil, castor oil, tall oil, and peanut oil; dibasic acid esters such as dibutyl phthalate and dioctyl phthalate; liquid rubbers such as polybutene and liquid isoprene rubber; and diethylene glycol, polyethylene glycol, propylene glycol, dipropylene glycol, and the like, and one of these may be used alone, or two or more may be used in combination. Among these, one or a combination of two or more selected from the group consisting of silicone oil, liquid paraffin, and liquid polybutene is preferable as the plasticizer. In the present invention, consider the case where these plasticizers are further contained in the adhesive agent layer. The content thereof is, in the case of two or more kinds, preferably 5 to 30% by mass, and more preferably 10 to 20% by mass in total relative to the total mass of the adhesive agent layer, from the viewpoint of improving the adhesive force of the adhesive agent layer and/or alleviating local irritation during release.

[Solubilizer · Filler]

Examples of the solubilizer include organic acids such as acetic acid, and surfactants, and one of these may be used alone, or two or more may be used in combination. In addition, the filler is blended mainly for the purpose of adjusting the adhesive force of the adhesive agent layer, and examples of the filler include aluminum hydroxide, calcium carbonate, and magnesium carbonate; silicates such as aluminum silicate and magnesium silicate; and silicic acid, barium sulfate, calcium sulfate, calcium zincate, zinc oxide, and titanium oxide, and one of these may be used alone, or two or more may be used in combination.

[Stabilizer]

Examples of the stabilizer include ascorbic acid or metal salts or esters thereof (preferably sodium salts and palmitate esters), isoascorbic acid or metal salts thereof (preferably sodium salts), ethylenediaminetetraacetic acid or metal salts thereof (preferably calcium disodium salts and tetrasodium salts), cysteine, acetylcysteine, dibutylhydroxytoluene, butylhydroxyanisole, propyl gallate, pentaerythrityl-tetrakis[3-(3,5-di-t-butyl-4-hydroxyphenyl)propionate], 3-mercapto-1,2-propanediol, tocopherol acetate, thymol, soy lecithin, rutin, dihydroxybenzoic acid, potassium dichloroisocyanurate, quercetin, hydroquinone, metal salts of hydroxymethanesulfinic acid (preferably sodium salts), metal metabisulfites (such as sodium salts), metal sulfites (preferably sodium salts), and metal thiosulfates (preferably sodium salt), and one of these may be used alone, or two or more may be used in combination. In the above, examples of the metal salts include sodium salts, potassium salts, calcium salts, and magnesium salts. In addition, examples of the esters include palmitate esters, stearate esters, and myristate esters.

In the present invention, consider the case where these stabilizers are further contained in the adhesive agent layer. The content thereof is, in the case of two or more kinds, preferably 10% by mass or less in total relative to the total mass of the adhesive agent layer.

[Preservative]

Examples of the preservative include derivatives of paraoxybenzoic acid, benzyl alcohol, phenol, cresol, and the like, and one of these may be used alone, or two or more may be used in combination.

Consider the case where the above additives are further contained in the adhesive agent layer. The content thereof is, in the case of two or more kinds, preferably 40% by mass or less, and more preferably 30% by mass or less in total relative to the total mass of the adhesive agent layer.

The adhesive agent layer according to the present invention is not particularly limited, but has a mass (thickness after production) per unit area (area of the sticking surface) of preferably 20 to 200 $g/m^2$, more preferably 30 to 100 $g/m^2$, and further preferably 30 to 70 $g/m^2$. In addition, the area (area after production) of the sticking surface of the adhesive agent layer according to the present invention can be appropriately adjusted depending on the purpose of treatment and the target of application, and is not particularly limited, but is usually in the range of 0.5 to 200 $cm^2$.

The rotigotine stabilization method of the present invention is a method comprising incorporating cross-linked polyvinylpyrrolidone in the adhesive agent layer of the rotigotine-containing patch in the predetermined amount described above.

The rotigotine-containing patch is not particularly limited, and can be produced by appropriately employing a known patch production method. For example, first, rotigotine and/or a pharmaceutically acceptable salt thereof, the styrene-based thermoplastic elastomer, and optionally a solvent and the additional components are kneaded in a usual manner to obtain a uniform adhesive agent layer composition. In the case of using a rotigotine free form as the rotigotine and/or the pharmaceutically acceptable salt thereof, the I-type crystals, II-type crystals, or amorphous form thereof may be used, or a mixture of at least two or more of the I-type crystals, II-type crystals, and amorphous form may be used. In addition, as the rotigotine and/or the pharmaceutically acceptable salt thereof, those dissolved in the solvent may be used. Examples of the solvent include anhydrous ethanol, toluene, heptane, methanol, ethyl acetate, hexane, a mixed solution of at least two or more of these, and the like.

Next, this adhesive agent layer composition is spread over the surface (usually over one surface) of the backing layer to a desired mass per unit area, and then the solvent is dried and removed by heating as necessary to form an adhesive agent layer, which is further cut into a desired shape as necessary. Thereby, it is possible to obtain the rotigotine-containing patch according to the present invention.

In addition, the method for producing the rotigotine-containing patch may further comprise a step of attaching the release liner to the surface of the adhesive agent layer opposite to the backing layer. The rotigotine-containing patch according to the present invention may be obtained by first spreading the adhesive agent layer composition over one surface of the release liner to a desired mass per unit area to form an adhesive agent layer, then attaching the backing layer to the surface of the adhesive agent layer opposite to the release liner, and cutting the unit into a desired shape as necessary. Moreover, the obtained patch may be enclosed in a preservation packaging container (such as an aluminum laminate bag) as necessary to form a package.

In the rotigotine stabilization method of the present invention, the method for incorporating cross-linked polyvinylpyrrolidone in the adhesive agent layer is not particularly limited. Examples of the method for producing the rotigotine-containing patch include a method comprising adding rotigotine and/or a pharmaceutically acceptable salt thereof, the styrene-based thermoplastic elastomer, an alicyclic saturated hydrocarbon resin, and optionally the solvent and the additional components such that the content of the cross-linked polyvinylpyrrolidone is the predetermined amount, and moreover optionally adding the antioxidant, the aliphatic alcohol, and the like and kneading them in a usual manner to obtain a uniform adhesive agent layer composition, and using this as the adhesive agent layer composition.

[Examples]

Hereinafter, the present invention is described more specifically based on Examples and Comparative Examples, but the present invention is not limited to the following Examples. Note that, in each of Examples and Comparative Examples, a skin permeation test and a stability evaluation were performed by the following methods.

<Skin Permeation Test (in vitro Hairless Mouse Skin Permeation Test)>

First, to the stratum corneum side of a fat-removed skin piece obtained by peeling the skin of a hairless mouse body and removing the fat, a patch cut into a square of 1.0 cm$^2$ with the release liner removed was attached. In this way, a test sample was prepared. This was set in a flow-through type diffusion cell such that the dermis side was in contact with a receptor solution, and the cell was filled with the receptor solution (phosphate buffered saline). Next, the receptor solution was sent at a flow rate of about 5 mL/hr while warm circulating water is circulated around the outer periphery so as to keep the receptor solution at 32°C, and the receptor solution was collected every 2 hours up to 24 hours. The concentration of the rotigotine in the collected receptor solution was measured by high performance liquid chromatography, and the following formula:

rate of skin permeation of rotigotine ($\mu$g/cm$^2$) = {concentration of rotigotine in receptor solution ($\mu$g/mL) $\times$ flow rate (mL)}/area of patch (cm$^2$)

was used calculate the rate of skin permeation of rotigotine per unit area of the adhesive agent layer, thereby obtaining the rate of skin permeation per hour (speed of skin permeation ($\mu$g/cm$^2$/hr)). The measurement was performed on each of two test samples, for each of which the average of the maximum values of speed of skin permeation within 24 hours was defined as the maximum speed of skin permeation (Jmax).

<Stability Evaluation>

The patch obtained in each of Examples and Comparative Examples was enclosed in an aluminum laminate bag to prepare a test sample, which was stored at 60°C for 2 weeks. To a solution obtained by removing the release liner from the patch after the storage and dissolving the adhesive agent layer in tetrahydrofuran, the following mobile phase liquid was added such that the total amount was 50 mL, and the mixture was filtered through a filter to prepare a test solution. The area of the peak region of the rotigotine in the test solution was figured out by using a high performance liquid chromatograph (manufactured by Shimadzu Corporation, column: ODS column, mobile phase liquid: mixed liquid of acetonitrile and a 0.1%-phosphate buffer containing 0.01 mol/L of sodium dodecyl sulfate (55:45), detection wavelength: 225 nm). In addition, for the test sample before the storage (immediately after being produced) too, the area of the peak region of the rotigotine in the test solution was figured out in a similar manner. Next, the following formula:

change in content of rotigotine [% by mass] = A/B $\times$ 100

[where A indicates the area of the peak region of the rotigotine in the test sample after the storage, and B indicates the area of the peak region of the rotigotine in the test sample before the storage]
was used to calculate the change in content of the rotigotine [% by mass] in the adhesive agent layer by the storage of the patch, and defined as the value of the stability evaluation. Note that the peak region appearing at a position around 6.5 was assumed as the peak region of the rotigotine.

(Example 1)

First, 3 parts by mass of cross-linked polyvinylpyrrolidone (cross-linked PVP) was added to 9.0 parts by mass of rotigotine (free form), 13.36 parts by mass of a styrene-isoprene-styrene block copolymer, 5.72 parts by mass of polyisobutylene, 48.66 parts by mass of an alicyclic saturated hydrocarbon resin, 15.26 parts by mass of a liquid paraffin, and 5 parts by mass of octyldodecanol, and they were added to an appropriate amount of a solvent (anhydrous ethanol and toluene) and mixed to obtain an adhesive agent layer composition. Next, the obtained adhesive agent layer composition was spread over a release liner (polyethylene terephthalate film subjected to release treatment), and the solvent was removed by drying to form an adhesive agent layer with a mass per unit area of 50 g/m$^2$. A backing layer (polyethylene terephthalate film) was stacked on the surface of the obtained adhesive agent layer opposite to the release liner to obtain a patch formed of a stack of backing layer/adhesive agent layer/release liner in this order.

(Examples 2 to 6 and Comparative Examples 1 to 3)

Each patch was obtained in the same manner as in Example 1 except that the constitution (excluding the solvent) of the adhesive agent layer composition was changed to the constitution shown in Table 1 below.

The patches obtained in Examples 1 to 6 and Comparative Examples 1 to 3 were subjected to a skin permeation test and a stability evaluation (2 weeks after production, 60°C). Table 1 shows the result of the stability evaluation together with the constitutions (excluding the solvent) of the adhesive agent layer compositions of Examples and Comparative Examples. Note that from the skin permeation test, it was observed that sufficiently good maximum speeds of skin permeation (Jmax) [$\mu g/cm^2/hr$] were achieved in Examples 1 to 6 (for example, 12.5 $\mu g/cm^2/hr$ in Example 6).

[Table 1]

|  | Comparative Example | | | Example | | | | | |
|---|---|---|---|---|---|---|---|---|---|
|  | 1 | 2 | 3 | 1 | 2 | 3 | 4 | 5 | 6 |
| Adhesive Agent Layer Composition [Parts by Mass] | | | | | | | | | |
| Rotigotine | 9 | 9 | 9 | 9 | 9 | 9 | 9 | 9 | 9 |
| Styrene-Isoprene-Styrene Block Copolymer | 13.84 | 14.82 | 14.77 | 13.36 | 13.31 | 13.03 | 13.65 | 13.63 | 13.62 |
| Polyisobutylene | 5.93 | 6.35 | 6.33 | 5.72 | 5.71 | 5.59 | 5.85 | 5.84 | 5.84 |
| Alicyclic Saturated Hydrocarbon Resin | 50.41 | 46.58 | 46.40 | 48.66 | 48.46 | 47.48 | 42.90 | 42.85 | 42.78 |
| Liquid Paraffin | 15.82 | 16.94 | 16.88 | 15.26 | 15.21 | 14.90 | 15.60 | 15.58 | 15.56 |
| Octyldodecanol | 5 | 5 | 5 | 5 | 5 | 5 | 3 | 3 | 3 |
| Cross-Linked PVP | - | 1 | 1 | 3 | 3 | 5 | 10 | 10 | 10 |
| 2-mercaptobenzimidazole | - | 0.31 | 0.62 | - | 0.31 | - | - | 0.1 | 0.2 |
| Total | 100 | 100 | 100 | 100 | 100 | 100 | 100 | 100 | 100 |
| Rotigotine : Cross-Linked PVP | - | 9:1 | 9:1 | 9:3 | 9:3 | 9:5 | 9:10 | 9:10 | 9:10 |
| Stability Evaluation (60°C, 2 Weeks) | 93.9 | 95.4 | 92.0 | 96.8 | 99.9 | 97.7 | 97.6 | 99.1 | 97.2 |

(Examples 7 to 9)

Each patch was obtained in the same manner as in Example 1 except that the constitution (excluding the solvent) of the adhesive agent layer composition was changed to the constitution shown in Table 2 below.
The patches obtained in Examples 7 to 9 were subjected to a stability evaluation (2 weeks after production, 60°C). Table 2 shows the result of the stability evaluation together with the constitutions (excluding the solvent) of the adhesive agent layer compositions of Examples.

[Table 2]

|  | Example | | |
|---|---|---|---|
|  | 7 | 8 | 9 |
| Adhesive Agent Layer Composition [Parts by Mass] | | | |
| Rotigotine | 13 | 15 | 9 |
| Styrene-Isoprene-Styrene Block Copolymer | 12.71 | 12.39 | 13.03 |
| Polyisobutylene | 5.45 | 5.31 | 5.59 |
| Alicyclic Saturated Hydrocarbon Resin | 46.31 | 45.14 | 47.48 |
| Liquid Paraffin | 14.53 | 14.16 | 14.90 |
| Octyldodecanol | 5 | 5 | - |

**EP 3 925 606 B1**

(continued)

|  | Example | | |
|---|---|---|---|
|  | 7 | 8 | 9 |
| Adhesive Agent Layer Composition [Parts by Mass] | | | |
| Cross-Linked PVP | 3 | 3 | 10 |
| Total | 100 | 100 | 100 |
| Rotigotine : Cross-Linked PVP | 13:3 | 15:3 | 9:10 |
| Stability Evaluation (60°C, 2 Weeks) | 100.0 | 99.7 | 96.8 |

As is clear from the results shown in Tables 1 and 2, for each of the patches in which predetermined amounts of cross-linked polyvinylpyrrolidone were incorporated in their adhesive agent layers (for example, Examples 1 to 9), it was observed that even after 2 weeks of being exposed to a temperature of 60°C, which was a harsh condition, after the production, the content of the rotigotine in the adhesive agent layer did not change from that immediately after the production and remained high, so that the rotigotine was stabilized at a high level. In addition, it was observed that better stability was exhibited when 2-mercaptobenzimidazole was further incorporated in the adhesive agent layer (for example, Examples 2, 5, and 6). Meanwhile, for the patches obtained in Comparative Examples 1 to 3, it was observed that the content of the rotigotine after the storage was sufficiently high but the stability thereof (for example, Comparative Example 1) was lower than those of the patches for which the rotigotine stabilization method of the present invention was carried out. It was observed that even when 2-mercaptobenzimidazole, which was an antioxidant, was further incorporated in the adhesive agent layer (for example, Comparative Examples 2 and 3), the stability of the rotigotine was not improved as much as those of the patches for which the rotigotine stabilization method of the present invention was carried out.

[Industrial Applicability]

As described above, according to the present invention, it is possible to provide a rotigotine stabilization method that enables high-level stabilization of rotigotine and/or a pharmaceutically acceptable salt thereof in an adhesive agent layer of a patch containing a styrene-isoprene-styrene block copolymer.

**Claims**

1. A rotigotine stabilization method for stabilizing rotigotine and/or a pharmaceutically acceptable salt thereof in a rotigotine-containing patch comprising a backing layer and an adhesive agent layer, the adhesive agent layer containing the rotigotine and/or the pharmaceutically acceptable salt thereof and a styrene-based thermoplastic elastomer,
the method comprising further incorporating cross-linked polyvinylpyrrolidone in a content of 3 to 25% by mass in the adhesive agent layer, and an alicyclic saturated hydrocarbon resin in the adhesive agent layer,
with the proviso that a patch prepared according to the following method is excluded:

to an appropriate amount of absolute ethanol and toluene the following components are added and mixed to obtain an adhesive agent layer composition,

| Adhesive agent layer composition [Parts by Mass] | | | | | | |
|---|---|---|---|---|---|---|
| Rotigotine | 9 | 9 | 9 | 9 | 9 | 9 |
| Styrene-Isoprene-Styrene Block Copolymer | 14.5 | 14.2 | 13.1 | 13.3 | 13.2 | 13.6 |
| Polyisobutylene | 6.2 | 6.1 | 5.7 | 5.7 | 5.6 | 5.9 |
| Alicyclic Saturated Hydrocarbon Resin | 45.7 | 44.5 | 42.2 | 41.55 | 41.45 | 42.70 |
| Liquid Paraffin | 16.6 | 16.2 | 15.0 | 15.2 | 15.1 | 15.5 |
| Octyldodecanol | 5 | 5 | 5 | 5 | 5 | 3 |
| Cross-Linked polyvinylpyrrolidone | 3 | 5 | 10 | 10 | 10 | 10 |
| Stabilizer (2-Mercaptobenz-imidazole) | - | - | - | 0.25 | 0.65 | 0.30 |

13

(continued)

| Adhesive agent layer composition [Parts by Mass] | | | | | | | |
|---|---|---|---|---|---|---|---|
| Rotigotine | 9 | 9 | 9 | 9 | 9 | 9 | 9 |
| Styrene-Isoprene-Styrene Block Copolymer | 13.3 | 13.3 | 13.3 | 13.3 | 13.3 | 13.2 | 12.9 |
| Polyisobutylene | 5.7 | 5.7 | 5.7 | 5.7 | 5.7 | 5.7 | 5.5 |
| Alicyclic Saturated Hydrocarbon Resin | 41.5 | 41.8 | 41.8 | 41.8 | 41.8 | 41.7 | 40.6 |
| Liquid Paraffin | 15.2 | 15.2 | 15.2 | 15.2 | 15.2 | 15.1 | 14.7 |
| Octyldodecanol | 5 | 5 | | | | 5 | 7 |
| Isostearyl alcohol | | | 5 | | | | |
| Hexyldecanol | | | | 5 | | | |
| Lauryl alcohol | | | | | 5 | | |
| Cross-Linked polyvinylpyrrolidone | 10 | 10 | 10 | 10 | 10 | 10 | 10 |
| Stabilizer (2-Mercaptobenz-imidazole) | 0.3 | - | - | - | - | 0.3 | 0.3 |

the obtained adhesive agent layer composition is applied on a polyethylene terephthalate film subjected to release treatment as a release liner, the solvent is removed by drying to a mass per unit area of 50 g/m$^2$ and the adhesive agent layer is formed thereby, a polyethylene terephthalate film as backing layer is stacked on the surface of the obtained adhesive agent layer opposite to the release liner to obtain a patch formed of a stack of backing layer/adhesive agent layer/release liner in this order.

2. The rotigotine stabilization method according to claim 1, wherein a content of the rotigotine and/or the pharmaceutically acceptable salt thereof in the adhesive agent layer is 5 to 15% by mass in terms of rotigotine free form.

3. The rotigotine stabilization method according to claim 1 or 2, wherein a mass ratio of a content of the rotigotine and/or the pharmaceutically acceptable salt thereof in terms of rotigotine free form and the content of the cross-linked polyvinylpyrrolidone in the adhesive agent layer (the content of the rotigotine and/or the pharmaceutically acceptable salt thereof in terms of rotigotine free form : the content of the cross-linked polyvinylpyrrolidone) is 15:3 to 5:25.

4. The rotigotine stabilization method according to any one of claims 1 to 3, further comprising further incorporating an imidazole-based antioxidant in the adhesive agent layer.

5. The rotigotine stabilization method according to claim 4, wherein a content of the imidazole-based antioxidant in the adhesive agent layer is 0.05 to 2% by mass.

6. The rotigotine stabilization method according to any one of claims 1 to 5, further comprising further incorporating an aliphatic alcohol in the adhesive agent layer.

**Patentansprüche**

1. Rotigotinstabilisierungsverfahren zur Stabilisierung von Rotigotin und/oder einem pharmazeutisch verträglichen Salz davon in einem Rotigotin-haltigen Pflaster, umfassend eine Trägerschicht und eine Klebstoffschicht, wobei die Klebstoffschicht das Rotigotin und/oder das pharmazeutisch verträgliche Salz davon und ein thermoplastisches Elastomer auf Styrolbasis enthält,
wobei das Verfahren ferner das Einbringen von vernetztem Polyvinylpyrrolidon in einem Gehalt von 3 bis 25 Massen-% in die Klebstoffschicht und eines alicyclischen gesättigten Kohlenwasserstoffharzes in die Klebstoffschicht umfasst,
mit der Maßgabe, dass ein nach dem folgenden Verfahren hergestelltes Pflaster ausgeschlossen ist:

zu einer geeigneten Menge an absolutem Ethanol und Toluol werden die folgenden Komponenten hinzugefügt und gemischt, um eine Klebstoffschichtzusammensetzung zu erhalten,

| Klebstoffschichtzusammensetzung [Massenanteile] | | | | | | |
|---|---|---|---|---|---|---|
| Rotigotin | 9 | 9 | 9 | 9 | 9 | 9 |
| Styrol-Isopren-Styrol-Blockcopolymer | 14,5 | 14,2 | 13,1 | 13,3 | 13,2 | 13,6 |
| Polyisobutylen | 6,2 | 6,1 | 5,7 | 5,7 | 5,6 | 5,9 |
| Alicyclisches gesättigtes Kohlenwasserstoffharz | 45,7 | 44,5 | 42,2 | 41,55 | 41,45 | 42,70 |
| Flüssiges Paraffin | 16,6 | 16,2 | 15,0 | 15,2 | 15,1 | 15,5 |
| Octyldodecanol | 5 | 5 | 5 | 5 | 5 | 3 |
| Vernetztes Polyvinylpyrrolidon | 3 | 5 | 10 | 10 | 10 | 10 |
| Stabilisator (2-Mercaptobenzimidazol) | - | - | - | 0,25 | 0,65 | 0,30 |

| Klebstoffschichtzusammensetzung [Massenanteile] | | | | | | | |
|---|---|---|---|---|---|---|---|
| Rotigotin | 9 | 9 | 9 | 9 | 9 | 9 | 9 |
| Styrol-Isopren-Styrol-Blockcopolymer | 13,3 | 13,3 | 13,3 | 13,3 | 13,3 | 13,2 | 12,9 |
| Polyisobutylen | 5,7 | 5,7 | 5,7 | 5,7 | 5,7 | 5,7 | 5,5 |
| Alicyclisches gesättigtes Kohlenwasserstoffharz | 41,5 | 41,8 | 41,8 | 41,8 | 41,8 | 41,7 | 40,6 |
| Flüssiges Paraffin | 15,2 | 15,2 | 15.2 | 15,2 | 15,2 | 15,1 | 14,7 |
| Octyldodecanol | 5 | 5 | | | | 5 | 7 |
| Isostearylalkohol | | | 5 | | | | |
| Hexyldecanol | | | | 5 | | | |
| Laurylalkohol | | | | | 5 | | |
| Vernetztes Polyvinylpyrrolidon | 10 | 10 | 10 | 10 | 10 | 10 | 10 |
| Stabilisator (2-Mercaptobenzimidazol) | 0,3 | - | - | - | - | 0,3 | 0,3 |

die erhaltene Klebstoffschichtzusammensetzung wird auf eine Polyethylenterephthalatfolie aufgebracht, die einer Trennbehandlung als Trennfolie unterzogen wurde, das Lösungsmittel wird durch Trocknen bis zu einer Masse pro Flächeneinheit von 50 g/m² entfernt und die Klebstoffschicht wird dadurch gebildet, eine Polyethylenterephthalatfolie wird als Trägerschicht auf die Oberfläche der erhaltenen Klebstoffschicht gegenüber der Trennfolie angeordnet, um ein Pflaster zu erhalten, das aus einer Anordnung von Trägerschicht/Klebstoffschicht/Trennfolie in dieser Reihenfolge gebildet ist.

2. Verfahren zur Stabilisierung von Rotigotin nach Anspruch 1, wobei der Gehalt an Rotigotin und/oder das pharmazeutisch verträgliche Salz davon in der Klebstoffschicht 5 bis 15 Massen-%, bezogen auf Rotigotin in freier Form, beträgt.

3. Verfahren zur Stabilisierung von Rotigotin nach Anspruch 1 oder 2, wobei das Massenverhältnis zwischen dem Gehalt an Rotigotin und/oder dem pharmazeutisch verträglichen Salz davon, bezogen auf Rotigotin in freier Form, und dem Gehalt an vernetztem Polyvinylpyrrolidon in der Klebstoffschicht (Gehalt an Rotigotin und/oder dem pharmazeutisch verträglichen Salz, bezogen auf Rotigotin in freier Form : Gehalt an vernetztem Polyvinylpyrrolidon) 15:3 bis 5:25 beträgt.

4. Verfahren zur Stabilisierung von Rotigotin nach einem der Ansprüche 1 bis 3, ferner umfassend das Einbringen eines Antioxidans auf Imidazolbasis in die Klebstoffschicht.

5. Verfahren zur Stabilisierung von Rotigotin nach Anspruch 4, wobei der Gehalt des Antioxidans auf Imidazolbasis in der Klebstoffschicht 0,05 bis 2 Massen-% beträgt.

6. Verfahren zur Stabilisierung von Rotigotin nach einem der Ansprüche 1 bis 5, ferner umfassend das Einbringen eines aliphatischen Alkohols in die Klebstoffschicht.

**Revendications**

1. Procédé de stabilisation de rotigotine pour stabiliser de la rotigotine et/ou son sel pharmaceutiquement acceptable dans un timbre contenant de la rotigotine comprenant une couche dorsale et une couche d'agent adhésif, la couche d'agent adhésif contenant la rotigotine et/ou son sel pharmaceutiquement acceptable et un élastomère thermoplastique à base de styrène,

   le procédé comprenant en outre l'incorporation de polyvinylpyrrolidone réticulée en une teneur de 3 à 25 % en masse dans la couche d'agent adhésif, et une résine hydrocarbonée saturée alicyclique dans la couche d'agent adhésif,
   à condition qu'un timbre préparé selon le procédé suivant soit exclu :

   à une quantité appropriée d'éthanol absolu et de toluène, les composants suivants sont ajoutés et mélangés pour obtenir une composition de couche d'agent adhésif,

| Composition de couche d'agent adhésif [parties en masse] | | | | | | |
|---|---|---|---|---|---|---|
| Rotigotine | 9 | 9 | 9 | 9 | 9 | 9 |
| Copolymère séquencé de styrène-isoprène-styrène | 14,5 | 14,2 | 13,1 | 13,3 | 13,2 | 13, 6 |
| Polyisobutylè ne | 6, 2 | 6, 1 | 5,7 | 5,7 | 5, 6 | 5, 9 |
| Résine hydrocarbonée saturée alicyclique | 45,7 | 44,5 | 42,2 | 41,55 | 41,45 | 42,70 |
| Paraffine liquide | 16, 6 | 16,2 | 15,0 | 15,2 | 15,1 | 15,5 |
| Octyldodécano l | 5 | 5 | 5 | 5 | 5 | 3 |
| Polyvinylpyrr olidone réticulée | 3 | 5 | 10 | 10 | 10 | 10 |
| Agent stabilisant (2-mercaptobenzimidazole) | - | - | - | 0,25 | 0, 65 | 0,30 |

| Composition de couche d'agent adhésif [parties en masse] | | | | | | | |
|---|---|---|---|---|---|---|---|
| Rotigotine | 9 | 9 | 9 | 9 | 9 | 9 | 9 |
| Copolymère séquencé de styrène-isoprène-styrène | 13,3 | 13,3 | 13,3 | 13,3 | 13,3 | 13,2 | 12, 9 |
| Polyisobutylè ne | 5,7 | 5,7 | 5,7 | 5,7 | 5,7 | 5,7 | 5,5 |
| Résine hydrocarbonée | 41,5 | 41,8 | 41,8 | 41,8 | 41,8 | 41,7 | 40, 6 |
| saturée alicyclique | | | | | | | |
| Paraffine liquide | 15,2 | 15,2 | 15,2 | 15,2 | 15,2 | 15,1 | 14, 7 |
| Octyldodécano l | 5 | 5 | | | | 5 | 7 |
| Alcool d'isostéaryle | | | 5 | | | | |
| Hexyldécanol | | | | 5 | | | |
| Alcool de lauryle | | | | | 5 | | |
| Polyvinylpyrr olidone réticulée | 10 | 10 | 10 | 10 | 10 | 10 | 10 |
| Agent stabilisant (2-mercaptobenzimidazole) | 0, 3 | - | - | - | - | 0, 3 | 0, 3 |

   la composition de couche d'agent adhésif obtenue est appliquée sur un film de poly(téréphtalate d'éthylène) soumise pour libérer un traitement comme protection antiadhésive, le solvant est retiré par séchage à une masse par surface unitaire de 50 g/m$^2$ et la couche d'agent adhésif est formée ainsi, un film de poly(téréphtalate d'éthylène) comme couche dorsale est empilé sur la surface de la couche d'agent adhésif obtenue opposée à la protection antiadhésive pour obtenir un timbre formé d'un empilement de couche dorsale/couche d'agent adhésif/protection antiadhésive dans cet ordre.

2. Procédé de stabilisation de rotigotine selon la revendication 1, dans lequel une teneur de la rotigotine et/ou de son sel pharmaceutiquement acceptable dans la couche d'agent adhésif est de 5 à 15 % en masse en termes de forme libre de rotigotine.

3. Procédé de stabilisation de rotigotine selon la revendication 1 ou 2, dans lequel un rapport en masse d'une teneur de la rotigotine et/ou de son sel pharmaceutiquement acceptable en termes de forme libre de rotigotine et la teneur de la polyvinylpyrrolidone réticulée dans la couche d'agent adhésif (la teneur de la rotigotine et/ou de son sel pharmaceutiquement acceptable en termes de forme libre de rotigotine:la teneur de la polyvinylpyrrolidone réticulée) est de 15:3 à 5:25.

4. Procédé de stabilisation de rotigotine selon l'une quelconque des revendications 1 à 3, comprenant en outre l'incorporation en outre d'un antioxydant à base d'imidazole dans la couche d'agent adhésif.

5. Procédé de stabilisation de rotigotine selon la revendication 4, dans lequel une teneur de l'antioxydant à base d'imidazole dans la couche d'agent adhésif est de 0,05 à 2 % en masse.

6. Procédé de stabilisation de rotigotine selon l'une quelconque des revendications 1 à 5, comprenant en outre l'incorporation en outre d'un alcool aliphatique dans la couche d'agent adhésif.

**REFERENCES CITED IN THE DESCRIPTION**

**Patent documents cited in the description**

- JP 2011504902 A
- JP 2002509878 A
- JP 2015503541 A
- JP 2014177428 A
- JP 2013079220 A
- JP 2006513195 A
- JP 2012504609 A
- JP 2013515041 A
- JP 2017515871 A
- WO 2012084969 A1